# EUROPEAN PATENT SPECIFICATION

(11) **EP 2 614 815 B1**
(45) Date of publication and mention of the grant of the patent: **16.01.2019**
(21) Application number: 11823670.2
(22) Date of filing: 09.09.2011
(51) Int. Cl.: A61K 9/12, A61K 31/045, A61K 31/07, A61K 31/618, A61K 47/34, A61P 29/00

(54) **FOAMABLE AEROSOL COMPOSITION**
SCHÄUMBARE AEROSOLZUSAMMENSETZUNG
COMPOSITION D'AÉROSOL MOUSSABLE

(30) Priority: 10.09.2010 JP 2010203546
(43) Date of publication of application: 17.07.2013
(73) Proprietor: Daizo Corporation, Osaka-shi Osaka 552-0013 (JP)
(72) Inventor: NAKAMOTO, Ryota, Kyoto-shi Kyoto 613-0916 (JP)
(74) Representative: Gille Hrabal
(86) International application number: PCT/JP2011/070603
(87) International publication number: WO 2012/033196

(56) References cited:
- EP-A1- 1 386 669
- WO-A1-90/11068
- WO-A1-91/01712
- WO-A1-2006/016549
- JP-A- 2 255 889
- JP-A- 4 103 526
- JP-A- 11 222 417
- JP-A- 62 054 784
- JP-A- 63 258 405
- JP-A- 2000 351 725
- JP-A- 2000 351 725
- JP-A- 2000 351 726
- JP-A- 2003 335 629
- JP-A- 2009 173 561
- JP-A- 2009 173 561
- JP-A- 2009 173 613
- US-A1- 2007 253 911

## Description

### TECHNICAL FIELD

The present invention relates to a foamable aerosol composition, in particular to a foamable aerosol composition containing a poorly water-soluble active ingredient at a high concentration.

### BACKGROUND ART

Various aerosol compositions containing an active ingredient at a high concentration and aerosol compositions forming foams emitting a cracking sound by breakage of foams have been proposed so far. For example, Patent Document 1 discloses an aerosol antiphlogistic anodyne comprising a concentrate containing 10 to 30 wt% of active ingredients comprising at least three of camphor, menthol, methyl salicylate and glycol salicylate. Patent Document 2 discloses a foam aerosol antiphlogistic anodyne preparation comprising 0.3 to 15 wt% of antiphlogistic anodyne, 0.1 to 5 wt% of ether surfactant, 3 to 20 wt% of water, 0.1 to 5 wt% of powder, 2.5 wt% or less of lower alcohol and 60 to 90 wt% of aerosol propellant. Patent Document 3 discloses an aerosol composition comprising an aqueous composition comprising aliphatic hydrocarbon having a boiling point of -5°C to 40°C and emitting a cracking sound by breakage of foams. Patent Document 4 discloses a foam aerosol preparation containing active ingredients in a base solution comprising 0.1 to 5 wt/vol% of surfactant, 0.05 to 10 wt/vol% of lower alcohol and/or glycols, 3 to 25 wt/vol% of water and 60 to 95 wt/vol% of n-butane gas. Further, Patent Document 5 discloses a cracking foam composition prepared by mixing an aerosol propellant and a concentrate in a concentrate-rich state. Patent Document 6 discloses a foamable aerosol composition comprising 0.1-10% of a single surfactant such as a nonionic surfactant, 0.01-30% active ingredients, water and alcohol, at least 38% propellants (LPG and DME). Patent Document 7 discloses a sherbet-like aerosol preparation having improved dustability, solubility, stability and percutaneous absorption of an active ingredient, extremely reduced skin irritation and excellent durability of refreshing feeling. The content of propellant is 60 to 99 % by weight. The composition of the document mainly uses dimethylether for forming the sherbet-like product, and only a small amount of liquefied pertroleum gas.. Patent Document 8 discloses a foamable aerosol composition with cooling effect. Example 5 discloses 35 g aqueous concentrate and 32.5 g liquefied petroleum gas.

### PRIOR ART DOCUMENTS

### Patent Documents

Patent Document 1: JP 1-020131 B
Patent Document 2: JP 7-078019 B
Patent Document 3: JP 2706693 B
Patent Document 4: JP 2741105 B
Patent Document 5: JP 3665925 B
Patent Document 6: JP 2003-335629 A
Patent Document 7: JP 2000-351725
Patent Document 8: JP 2009-173561 A

### SUMMARY OF THE INVENTION

### PROBLEM TO BE SOLVED BY THE INVENTION

However, an aerosol antiphlogistic anodyne of Patent Document 1 is a spray type aerosol antiphlogistic anodyne to be sprayed in the form of fine fog drops, and since aspiration by a user is easy and especially active ingredients are blended at a high concentration, there is a problem that a user is choked, which is inconvenient to the user. In a foam aerosol antiphlogistic anodyne preparation of Patent Document 2, there is no problem with aspiration, but freon is used as an aerosol propellant, and since a liquid density of freon is larger than that of water, stability is largely different as compared with the case of using aliphatic hydrocarbon (liquefied petroleum gas). In Patent Documents 3 and 4, since foams are formed, there is no problem with aspiration, but when aliphatic hydrocarbon is used as an aerosol propellant and active ingredients are blended at a high concentration, stability is lowered and a uniform composition cannot be sprayed. In Patent Document 5, too, there is no problem with aspiration because foams are formed, but the composition is in a concentrate-rich state and an amount of the aerosol propellant is small, and therefore, a density of foams is high and there is a problem that in applying and spreading the composition on an object to be coated, dripping of the composition occurs easily.

The present invention was made in the light of the above-mentioned problems, and an object of the present invention is to provide a foamable aerosol composition, in which though poorly water-soluble active ingredients are blended at a high concentration, a concentrate containing water can be used by using a specific surfactant and a poorly water-soluble liquefied gas, emulsification stability of the concentrate and the liquefied gas is good, and a uniform composition can be sprayed.

### MEANS TO SOLVE THE PROBLEM

The foamable aerosol composition of the present invention is one comprising a concentrate and a liquefied gas, in which the concentrate comprises active ingredients, surfactants, a monovalent alcohol having 2 to 3 carbon atoms and water, the surfactants comprise polyoxyethylene-added fatty acid ester and a nonionic surfactant having HLB of from 8 to 19, wherein the polyoxyethylene-added fatty acid ester is polyoxyethylene sorbitan fatty acid ester, and the nonionic surfactant is polyoxyethylene-hydrogenated castor oil and/or an ether surfactant, the liquefied gas is a poorly water-soluble liquefied gas having solubility in water at 20 °C of less than 10 wt%, the liquefied gas contains aliphatic hydrocarbon in an amount of 60 wt% or more, the ratio of the concentrate to the liquefied gas is 60/40 to 10/90 (weight ratio), the amount of poorly water-soluble active ingredients is from 5 to 30 wt% in the concentrate, and the concentrate and the liquefied gas are emulsified.

It is preferable that the active ingredients are poorly water-soluble active ingredients having solubility in water at 25°C of less than 10 wt%.

The amount of the poorly water-soluble active ingredients is from 5 to 30 wt% in the concentrate.

It is preferable that the poorly water-soluble active ingredients are antiphlogistic anodynes.

It is preferable that the amount of the monovalent alcohol having 2 to 3 carbon atoms is from 5 to 40 wt% in the concentrate.

It is preferable that the amount of water is from 40 to 85 wt% in the concentrate.

The polyoxyethylene-added fatty acid ester is polyoxyethylene sorbitan fatty acid ester.

The nonionic surfactant is polyoxyethylene hydrogenated castor oil and/or an ether surfactant.

### EFFECT OF THE INVENTION

According to the present invention, it is possible to provide the foamable aerosol composition, in which though poorly water-soluble active ingredients are blended at a high concentration, a concentrate containing water can be used by dissolving the active ingredients in alcohol and using a specific surfactant and a poorly water-soluble liquefied gas, and since emulsification stability of the concentrate and the liquefied gas is good, a uniform composition can be sprayed even if active ingredients being hardly soluble in water are used. Further, it is possible to provide the foamable aerosol composition, in which while foams are formed upon spraying of the foamable aerosol composition, by blending the liquefied gas in a large amount, dripping of the composition hardly occurs in coating and spreading the composition on an object to be coated such as a skin, and further, good feeling of coolness can be obtained due to cooling by heat of evaporation.

### EMBODIMENT FOR CARRYING OUT THE INVENTION

The foamable aerosol composition of the present invention is defined in claim 1 an is one comprising a concentrate and a liquefied gas, in which the concentrate comprises active ingredients having a solubility in water as defined in claim 1, surfactants, a monovalent alcohol having 2 to 3 carbon atoms and water, the surfactants comprise polyoxyethylene-added fatty acid ester and a nonionic surfactant having HLB of from 8 to 19 as specified in claim 1, the liquefied gas is a poorly water-soluble liquefied gas, the ratio of the concentrate to the liquefied gas is 60/40 to 10/90 (weight ratio), and the concentrate and the liquefied gas are emulsified.

The above-mentioned active ingredients exhibit their effect when applied to a human body, and though the foamable aerosol composition of the present invention easily adheres to and infiltrated into a skin, and poorly water-soluble active ingredients which are hardly dissolved in water can be used. The above-mentioned poorly water-soluble active ingredients are dissolved in alcohol and emulsified with aqueous components in a state of being contained in the concentrate, and further, is emulsified with the liquefied gas together with aqueous components even in a state of being contained in the aerosol composition. Therefore, the aerosol composition is easily sprayed in a state of the active ingredients being dispersed at a uniform concentration, and further, since the active ingredients easily keep an emulsified state with the aqueous components even after the aerosol composition is sprayed outside and the liquefied gas is evaporated, the aerosol composition can be uniformly applied.

Here, the poorly water-soluble active ingredients are active ingredients having solubility in water at 25°C of less than 10 wt%, preferably less than 5 wt%.

Examples of the active ingredient are antiphlogistic anodynes such as methyl salicylate, indomethacin, felbinac and ketoprofen; refrigerants such as 1-menthol and camphor; ultraviolet absorbers such as p-methoxy cinnamic acid ethylhexyl, p-methoxy cinnamic acid isopropyl, p-methoxy cinnamic acid octyl, diethylamino hydroxybenzoyl hexyl benzoate, t-butyl methoxybenzoyl methane, ethylhexyl triazone, octocrylene, oxybenzone, hydroxybenzophenone sulfonic acid, sodium hydroxybenzophenone sulfonate, dihydroxybenzophenone and 4-aminobenzoic acid; vermin repellents such as N,N-diethyl-m-toluamide (Deet); antioxidants such as α-tocopherol and dibutylhydroxytoluene; vitamins such as retinol and dl-α-tocopherol; anti-inflammatory agents such as glycyrrhetinic acid; antifungal agents such as miconazole nitrate, sulconazole nitrate and clotrimazole; deodorants and perfumes such as lauryl methacrylate, geranyl crotonate, acetophenone myristate, benzyl acetate, benzyl propionate and methyl phenylacetate; and the like.

The amount of the above-mentioned poorly water-soluble active ingredients is from 5 to 30 wt%, preferably from 8 to 25 wt% in the concentrate. When the amount of the above-mentioned poorly water-soluble active ingredients is less than 5 wt%, effect of the active ingredients is apt to be insufficient, and when more than 30 wt%, emulsification stability is not good and a uniform composition is hardly sprayed.

The above-mentioned poorly water-soluble active ingredients are blended as an alcohol solution in a state of being dissolved in the monovalent alcohol having 2 to 3 carbon atoms. Examples of the alcohol are ethanol, isopropanol and the like.

The amount of the above-mentioned monovalent alcohol having 2 to 3 carbon atoms is preferably from 5 to 40 wt%, further preferably from 10 to 35 wt%, especially preferably from 17 to 30 wt% in the concentrate. When the amount of the above-mentioned monovalent alcohol having 2 to 3 carbon atoms is less than 5 wt%, the poorly water-soluble active ingredients are hardly dissolved therein at a high concentration, and when more than 40 wt%, emulsification hardly occurs, and even if emulsification arises, emulsification stability is not good and a uniform composition is hardly sprayed.

The concentration of the above-mentioned poorly water-soluble active ingredients in the alcohol solution is preferably from 15 to 70 wt%, further preferably from 20 to 50 wt%. When the concentration is less than 15 wt%, the amount of the active ingredients which can be blended is small, and when more than 70 wt%, there is a case where the active ingredients are precipitated without being dissolved.

The surfactants in a state of being contained in the concentrate act to emulsify the alcohol solution containing the poorly water-soluble active ingredients with water, and further act to emulsify the concentrate with the liquefied gas in a state of being contained in the aerosol composition, thereby forming foams upon spraying of the aerosol composition to the outside.

The surfactants comprising polyoxyethylene-added fatty acid ester and a nonionic surfactant having HLB of from 8 to 19 are used from the viewpoint that emulsification stability of the concentrate and the aerosol composition is good even when the poorly water-soluble active ingredients are contained at a high concentration and a uniform composition is easily sprayed, that foams of fine texture are formed though the liquefied gas is contained in a large amount, and that the foams are defoamed immediately upon applying on a skin and are easily spread thereon.

The polyoxyethylene-added fatty acid ester are polyoxyethylene sorbitan fatty acid esters such as POE sorbitan monolaurate, POE sorbitan monostearate and POE sorbitan monooleate In the present invention, polyoxyethylene hydrogenated castor oil as mentioned infra is not included in the polyoxyethylene-added fatty acid esters.

Particularly from the viewpoint that emulsification stability of the concentrate and the aerosol composition is good and the concentrate and the aerosol composition are easily re-emulsified even when they are separated from each other in the aerosol composition, polyoxyethylene-added fatty acid ester having HLB of from 9 to 18, preferably from 10 to 17, namely polyoxyethylene sorbitan fatty acid ester is used.

Examples of the nonionic surfactant are ether surfactants, for example, polyoxyethylene alkyl ethers such as POE cetyl ether, POE stearyl ether, POE oleyl ether, POE lauryl ether, POE behenyl ether, POE octyl dodecyl ether, POE isocetyl ether and POE isostearyl ether, and polyoxyethylene polyoxypropylene alkyl ethers such as POE POP cetyl ether and POE POP decyl tetradecyl ether; polyoxyethylene hydrogenated castor oil, and a mixture thereof. In addition, the above-mentioned polyoxyethylene-added fatty acid esters are not included in the nonionic surfactant in the present invention.

Particularly from the viewpoint that emulsification stability of the concentrate and the aerosol composition is good and the concentrate and the aerosol composition are easily re-emulsified even when they are separated from each other in the aerosol composition, polyoxyethylene hydrogenated castor oil and/or ether surfactant are used.

The HLB of the nonionic surfactant is from 8 to 19, further preferably from 9 to 17. When the HLB of the nonionic surfactant is less than 8, there is a tendency that the concentrate and the aerosol composition are hardly emulsified and even if they are emulsified, immediately separation occurs. When more than 19, there is a tendency that defoaming hardly occurs even after applying on a skin because foaming of the sprayed composition is too good.

Particularly from the viewpoint that emulsification stability of the concentrate and the aerosol composition is good and a uniform composition is easily sprayed, polyoxyethylene sorbitan fatty acid esters having HLB of from 9 to 18 as the polyoxyethylene-added fatty acid ester, and polyoxyethylene hydrogenated castor oil and/or polyoxyethylene alkyl ether as the nonionic surfactant having HLB of from 8 to 19 are used.

The amount of the above-mentioned surfactant is preferably from 0.5 to 15 wt%, further preferably from 1 to 10 wt% in the concentrate. When the amount of the surfactant is less than 0.5 wt%, there is a tendency that emulsification hardly occurs in the concentrate and the aerosol composition, and even if emulsification arises, emulsification stability is not good and a uniform composition is hardly sprayed, and when the amount of the surfactant is more than 15 wt%, the surfactants are apt to remain on a skin and impression from use is not good due to sticking and the like.

The above-mentioned water is a main solvent of the concentrate and is emulsified with the alcohol solution containing the poorly water-soluble active ingredients and the liquefied gas. When the aerosol composition containing water is sprayed, foaming arises and a liquid film is formed. Examples of water are purified water, ion-exchanged water and the like.

The amount of the above-mentioned water is preferably from 40 to 85 wt%, further preferably from 50 to 80 wt%, especially preferably from 50 to 70 wt% in the concentrate. When the amount of water is less than 40 wt%, there is a tendency that emulsification hardly occurs, and even if emulsification arises, emulsification stability is not good and a uniform composition cannot be sprayed, and when the amount of water is more than 85 wt%, it becomes difficult to blend the poorly water-soluble active ingredients in an amount of 5 wt% or more.

In addition to the above-mentioned poorly water-soluble active ingredients, mono-valent alcohols having 2 to 3 carbon atoms, surfactants and water, a water-soluble active ingredient, a water-soluble polymer, a pH regulator, oil, powder and the like can be blended to the concentrate.

The water-soluble active ingredient is blended to the aqueous component and is used for the purposes of assisting the poorly water-soluble active ingredients in exhibiting the effects thereof or imparting another effect.

Examples of the above-mentioned water-soluble active ingredient are humectants such as ethylene glycol, propylene glycol, glycerin, 1,3-butylene glycol, collagen, xylitol, sorbitol, hyaluronic acid, karonin acid, sodium lactate, dl-pyrrolidone carboxylate, keratin, casein, lecithin and urea; sterilization and disinfection agents such as paraoxybenzoic acid, sodium benzoate, potassium sorbate, phenoxyethanol, benzalkonium chloride, benzethonium chloride, chlorhexidine chloride, photosensitive material and p-chlorometacresol; amino acids such as glycine, alanine, leucine, serine, tryptophane, cystine, cysteine, methionine, aspartic acid, glutamic acid and arginine; vitamins such as calcium pantothenate, ascorbic acid magnesium phosphate and sodium ascorbate, water soluble perfumes, and the like.

The amount of the above-mentioned water-soluble active ingredient is preferably from 0.01 to 10 wt%, further preferably from 0.1 to 5 wt% in the concentrate. When the amount of the water-soluble active ingredient is less than 0.01 wt%, the effect of the water-soluble active ingredient is hardly obtained, and when the amount of the water-soluble active ingredient is more than 10 wt%, there is a case where emulsification is inhibited.

The above-mentioned water-soluble polymer is used for the purpose of increasing a viscosity of the concentrate to adjust foaming and defoaming conditions. Examples of the above-mentioned water-soluble polymer are cellulose polymers such as hydroxyethyl cellulose, hydroxypropyl cellulose, hydroxypropyl methyl cellulose and carboxymethyl-cellulose sodium; gums such as xanthan gum, carrageenan, acacia gum, tragacanth gum, cationic guar gum, guar gum, gellan gum and locust bean gum; dextran, carboxymethyl-dextran sodium, dextrine, pectin, starch, corn starch, wheat starch, sodium alginate, denatured potato starch, hyaluronic acid, sodium hyaluronate, polyvinyl alcohol, polyvinyl pyrrolidone, carboxyvinyl polymer and the like. Especially, from the viewpoint that the power of holding the liquefied gas in the concentrate is increased and a foam-cracking sound and feeling of cracking are easily obtained when the foams are broken, it is preferable to use cellulose polymers, gums, especially hydroxyethyl cellulose and carrageenan.

The amount of the above-mentioned water-soluble polymer is preferably from 0.01 to 2 wt%, further preferably from 0.05 to 1 wt% in the concentrate. When the amount of the water-soluble polymer is less than 0.01 wt%, the effect of the water-soluble polymer is hardly obtained, and when the amount of the water-soluble polymer exceeds 2 wt%, the viscosity becomes too high and emulsification hardly arises.

The above-mentioned pH regulator is used for the purposes of adjusting a pH value of the concentrate to make the poorly water-soluble active ingredients to be easily dissolved and neutralizing an association type water-soluble polymer such as carboxyvinyl polymer to adjust the viscosity.

Examples of the above-mentioned pH regulator are organic alkali such as triethanolamine (TEA), diethanolamine (DEA), monoethanolamine (MEA), diisopropanolamine (DIPA), 2-amino-2-methyl-1-propanol (AMP) and 2-amino-2-methyl-1,3-propanediol (AMPD), inorganic alkali such as potassium hydroxide, sodium hydroxide and ammonium hydroxide, organic acids such as citric acid, glycolic acid, lactic acid and phosphoric acid, inorganic acids such as hydrochloric acid, and the like.

When the pH regulator is blended, it is preferable to blend it so that the pH value of the concentrate becomes 4 to 10, further 5 to 9. When the pH value of the concentrate is lower than 4, an aerosol container tends to be corroded easily, and when the pH value is higher than 10, there is a tendency that there easily arises an influence such as stimulus on a skin.

The above-mentioned oil is used for the purpose of improving impression from use, i.e., giving feeling of wetness on a skin which is easily subject to degreasing due to alcohol.

Examples of the oil are fats and fatty oils such as meadowfoam seed oil, olive oil, camellia oil, corn oil, castor oil, safflower oil, jojoba oil and coconut oil; unsaturated aliphatic acids such as eicosenoic acid and oleic acid; higher alcohols such as oleyl alcohol and isostearyl alcohol; ester oils such as isopropyl myristate, isopropyl palmitate, cetyl isooctanoate, octyl hydroxystearate, ethylhexyl hydroxystearate, methyl pentanediol dineopentanoate, diethyl pentanediol dineopentanoate, neopentyl glycol di-2-ethylhexanoate, neopentyl glycol dicaprate, propylene glycol dilaurate, ethylene glycol distearate, diethylene glycol dilaurate, diethylene glycol distearate, diethylene glycol diisostearate, diethylene glycol dioleate, triethylene glycol dilaurate, triethylene glycol distearate, triethylene glycol diisostearate, triethylene glycol dioleate, propylene glycol monostearate, propylene glycol monooleate, ethylene glycol monostearate, glyceryl tri-2-ethylhexanoate, caprylic/capric triglyceride, ethylhexyl methoxy cinnamate, isononyl isononanoate, isotridecyl isononanoate, diethoxyethyl succinate and diisostearyl malate; silicone oils such as methyl polysiloxane, cyclopentasiloxane, cyclohexasiloxane, octamethylcyclotetrasiloxane, decamethylcyclopentasiloxane, dodecamethylcyclohexasiloxane, methylcyclopolysiloxane, tetrahydrotetramethylcyclotetrasiloxane, octamethyltrisiloxane, decamethyltetrasiloxane, methylhydrogene polysiloxane and methylphenyl polysiloxane; hydrocarbons such as liquid paraffin and isoparaffin, and a mixture thereof.

When the above-mentioned oil is blended, the amount thereof is preferably from 0.1 to 15 wt%, further preferably from 0.5 to 12 wt% in the concentrate. When the amount of the oil is less than 0.1 wt%, the effect of the oil is hardly obtained, and when the amount of the oil exceeds 15 wt%, impression from use is apt to be lowered, i.e., drying characteristic is lowered and sprayed foams are sticky.

The above-mentioned powder is used for the purpose of making emulsification of the concentrate and the aerosol composition easy and imparting feeling of dryness. Examples of the powder are talc, silica, zinc oxide, titanium oxide, zeolite, kaolin, mica, magnesium carbonate, calcium carbonate, magnesium silicate, aluminum silicate, ceramic powder, silicon powder, nylon powder and the like. Powders in the spherical form may be used.

When the above-mentioned powder is blended, the amount thereof is preferably from 0.01 to 10 wt%, further preferably from 0.05 to 5 wt% in the concentrate. When the amount of the powder is less than 0.01 wt%, the effect of the powder is hardly obtained, and when the amount of the powder exceeds 10 wt%, caking of the powder easily occurs at the bottom of the aerosol container and a valve and an orifice of an actuator are clogged with the powder.

The above-mentioned concentrate can be prepared by mixing an alcohol solution obtained by dissolving the poorly water-soluble active ingredients in the monovalent alcohol having 2 to 3 carbon atoms with an aqueous solution obtained by blending the surfactant in water, followed by emulsification. The surfactant may be blended in the monovalent alcohol or in water. In the case of blending a powder, it may be dispersed in the concentrate or the powder may be filled in a container beforehand separately from the concentrate.

The amount of the concentrate is preferably from 10 to 60 wt%, further preferably from 20 to 50 wt% in the aerosol solution. When the amount of the concentrate is less than 10 wt%, the concentrate is hardly emulsified with the liquefied gas, and when the amount of the concentrate exceeds 60 wt%, a density of the foams is high and the foams are apt to drip when applied and spread on a skin.

The above-mentioned liquefied gas is a liquid having a vapor pressure in the aerosol container, has a low solubility in water, is emulsified with the concentrate containing a large amount of water to form the aerosol composition, and after sprayed to the outside, is vaporized to form foams.

In the present invention, the liquefied gas to be blended is characterized by being a poorly water-soluble liquefied gas having solubility in water at 20°C of less than 10 wt%, preferably less than 5 wt%. When the solubility of the liquefied gas in water is more than 10 wt%, the liquefied gas is hardly emulsified with the concentrate and emulsification stability is apt to be lowered, and therefore, a uniform composition tends to be hardly sprayed. In addition, in the case of blending a gas mixture as the liquefied gas, it is enough for the gas mixture to have properties corresponding to those of a poorly water-soluble liquefied gas.

Examples of the above-mentioned liquefied gas are aliphatic hydrocarbons having 3 to 5 carbon atoms such as propane, butane, pentane and a mixture thereof, hydrofluoroolefin such as trans-1,3,3,3-tetrafluoroprop-1-ene, hydrofluorocarbon such as 1,1-difluoroethane and a mixture thereof. In addition, from the viewpoint of easy emulsification with the concentrate and good emulsification stability, aliphatic hydrocarbon is blended in the liquefied gas in an amount of 60 wt% or more, preferably 70 wt% or more.

To the above-mentioned liquefied gas can be blended dimethyl ether being a water soluble liquefied gas within the above-mentioned solubility range. By blending dimethyl ether, cooling effect can be increased. In the case of blending dimethyl ether, the amount thereof is preferably 40 wt% or less, further preferably 30 wt% or less in the liquefied gas. When the amount of dimethyl ether is more than 40 wt%, solubility in water is increased and dimethyl ether is hardly emulsified with the concentrate and a uniform composition is hardly sprayed.

The amount of the above-mentioned liquefied gas is preferably from 40 to 90 wt%, further preferably from 50 to 80 wt% in the aerosol composition. When the amount of the liquefied gas is less than 40 wt%, a density of the foams is high and the foams are apt to drip when applied and spread on a skin. Further, the foams are hardly broken in such a manner as being cracked and feeling of coolness is decreased. Meanwhile, when more than 90 wt%, the liquefied gas is hardly emulsified with the concentrate and a uniform composition is hardly sprayed.

The above-mentioned foamable aerosol composition can be prepared by filling the concentrate and the liquefied gas in a pressure resistant container and fixing an aerosol valve to the pressure resistant container, thereby sealing the aerosol container. In addition, the liquefied gas may be filled through the aerosol valve after fixing the valve to the container, or may be filled by an under-cup filling method immediately before fixing an aerosol valve. Further, by mounting a spray member on the aerosol valve, an aerosol product is obtained.

A pressure resistant container made of a resin such as polyethylene terephthalate, a pressure resistant container made of glass, or a pressure resistant container made of metal such as aluminum or tinplate can be used as the pressure resistant container.

Moreover, compressed gas such as carbon dioxide gas, nitrogen gas, compressed air or oxygen gas can be used as a pressurizing gas for adjusting the pressure of the foamable aerosol composition.

In the above-mentioned foamable aerosol composition, the concentrate and the liquefied gas are filled in the pressure resistant container, and are mixed by shaking or the like of the container to emulsify the concentrate and the liquefied gas and form an oil-in-water type emulsion. In the foamable aerosol composition, though the poorly water-soluble active ingredients are contained in the concentrate at a concentration being as high as 5 to 30 wt%, the active ingredients can be dissolved in alcohol, and by using the specific surfactant with the poorly water-soluble liquefied gas, the concentrate containing water can be used, and further, a uniform composition can be sprayed since emulsification stability of the concentrate and the liquefied gas is good. Furthermore, while the sprayed aerosol composition is foamed to form foams, since the liquefied gas is contained in a large amount of from 40 to 90 wt%, the foams are cooled due to heat of evaporation and by spreading the foams on a skin, good feeling of coolness can be obtained.

The present invention is specifically explained below by means of Examples, but is not limited thereto.

Methods for evaluation are described below.

### 1) Easiness of emulsification

A container is subjected to shaking up and down, and the number of shakes until emulsification is completed is determined.
⊚: Emulsification occurs by shaking 10 times or less.
○: Emulsification occurs by shaking 11 to 20 times.
Δ: Emulsification occurs by shaking 21 to 30 times.
×: No emulsification occurs by shaking 30 times.

### 2) Emulsification stability

After emulsification, a container is left at rest at a room temperature, and a time period until separation occurs is measured.
⊚: No separation occurs for 30 days or more.
○: Separation starts in 15 days.
Δ: Separation starts within 7 days.
×: Separation occurs within one day.

### 3) Impression from use

A container is allowed to stand in a 25°C thermostatic water chamber for one hour, and then after spraying and spreading 0.5 g of an aerosol composition on an arm, condition of the sprayed composition is evaluated.
⊚: A large foam-cracking sound is emitted and an aerosol composition can be uniformly applied and spread without dripping.
○: A foam-cracking sound is emitted and an aerosol composition can be uniformly applied and spread without dripping.
Δ: A small foam-cracking sound is emitted and dripping partly occurs when spreading.
×1: No foam-cracking sound is emitted and dripping occurs when spreading.
X2: An aerosol composition is scattered without forming foams.

### 4) Feeling of coolness

A container is allowed to stand in a 25°C thermostatic water chamber for one hour, and then after spraying and spreading 0.5 g of an aerosol composition on an arm, feeling of coolness is evaluated.
⊚: Very good comfortable cooling effect is obtained.
○: Cooling effect is obtained.
×: Cooling effect is not obtained.
-: Not evaluated as no application is properly made.

### EXAMPLE

### EXAMPLES 1 to 11

The concentrates shown in Table 1 were prepared, each solution was filled in a transparent pressure resistant polyethylene terephthalate container in an amount of 20 g (40 wt%), and an aerosol valve was fixed to the container. Then, a liquefied petroleum gas (*1) was filled as a liquefied gas in amount of 30 g (60 wt%), and the container was shaken up and down for emulsification of the concentrate and the liquefied gas to prepare an aerosol product filled with an aerosol composition for resolution of inflammation and alleviation of pain. Easiness of emulsification, emulsification stability, impression from use and feeling of coolness of the obtained aerosol product were evaluated. The results of evaluation are shown in Table 2.

**TABLE 1**

| | Example | | | | | | | | | | |
|---|---|---|---|---|---|---|---|---|---|---|---|
| | 1 | 2 | 3 | 4 | 5 | 6 | 7 | 8 | 9 | 10 | 11 |
| Methyl salicylate | 10.0 | 10.0 | 10.0 | 10.0 | 10.0 | 10.0 | 10.0 | 10.0 | 12.0 | 15.0 | 5.0 |
| 1-Menthol | 3.0 | 3.0 | 3.0 | 3.0 | 3.0 | 3.0 | 3.0 | 3.0 | 4.0 | 5.0 | 1.0 |
| POE(20) sorbitan monolaurate (*2) | 2.0 | 2.0 | 2.0 | - | - | 2.0 | 3.0 | 2.0 | 2.0 | 2.0 | 2.0 |
| POE(60) sorbitol tetraoleate (*3) | - | - | - | 2.0 | 2.0 | - | - | - | - | - | - |
| POE(40) hydrogenated castor oil (*4) | 1.0 | 1.0 | 1.0 | 1.0 | 1.0 | 1.0 | 1.5 | 1.0 | 1.0 | 1.0 | 1.0 |
| POE(2) lauryl ether (*5) | 0.5 | 0.5 | 0.5 | 0.5 | 0.5 | 0.5 | 0.7 | 0.5 | 0.5 | 0.5 | 0.5 |
| POE(20) cetyl ether (*6) | - | 0.5 | 0.5 | - | 0.5 | 0.5 | 0.7 | 0.5 | 0.5 | 0.5 | 0.5 |
| Hydroxyethyl cellulose (*7) | - | - | 0.1 | - | 0.1 | 0.1 | 0.1 | 0.1 | 0.1 | 0.1 | 0.1 |
| Ethanol | 20.0 | 20.0 | 20.0 | 20.0 | 20.0 | 17.0 | 30.0 | 20.0 | 20.0 | 20.0 | 20.0 |
| Purified water | 62.5 | 62.0 | 61.9 | 62.5 | 61.9 | 64.9 | 50.0 | 61.9 | 58.9 | 54.9 | 68.9 |
| Talc | 1.0 | 1.0 | 1.0 | 1.0 | 1.0 | 1.0 | 1.0 | 0.5 | 1.0 | 1.0 | 1.0 |
| Spherical silica (*8) | - | - | - | - | - | - | - | 0.5 | - | - | - |
| Total | 100.0 | 100.0 | 100.0 | 100.0 | 100.0 | 100.0 | 100.0 | 100.0 | 100.0 | 100.0 | 100.0 |

| | | | | | | | | | | | |
|---|---|---|---|---|---|---|---|---|---|---|---|
| *1: Vapor pressure at 20°C: 0.15 MPa, solubility in water at 20°C: 1 wt% or less, a mixture of normal butane and isobutane *2: NIKKOL TL-10 (trade name), HLB: 16.9, available from Nikko Chemicals Co., Ltd. *3: NIKKOL GO-460V (trade name), HLB: 14.0, available from Nikko Chemicals Co., Ltd. *4: NIKKOL HCO-40 (trade name), HLB: 12.5, available from Nikko Chemicals Co., Ltd. *5: NIKKOL BL-2 (trade name), HLB: 9.5, available from Nikko Chemicals Co., Ltd. *6: NIKKOL BC-20TX (trade name), HLB: 17.0, available from Nikko Chemicals Co., Ltd. *7: HEC-SE850 (trade name) available from Nitta Gelatin Inc. *8: Sylosphere C-1504 (trade name) available from FUJI SILYSIA CHEMICAL LTD. | | | | | | | | | | | |

| | |
|---|---|
| Concentrate | 40.0 |
| Liquefied petroleum gas (*1) | 60.0 |
| Total | 100.0 wt% |

### EXAMPLE 12

An aerosol product filled with an aerosol composition for resolution of inflammation and alleviation of pain was prepared in the same manner as in Example 1 except that 25 g (50 wt%) of the concentrate of Example 3 and 25 g (50 wt%) of the liquefied petroleum gas (*1) as the liquefied gas were used. Easiness of emulsification, emulsification stability, impression from use and feeling of coolness of the obtained aerosol product were evaluated. The results of evaluation are shown in Table 2.

### EXAMPLE 13

An aerosol product filled with an aerosol composition for resolution of inflammation and alleviation of pain was prepared in the same manner as in Example 1 except that 30 g (60 wt%) of the concentrate of Example 3 and 20 g (40 wt%) of the liquefied petroleum gas (*1) as the liquefied gas were used. Easiness of emulsification, emulsification stability, impression from use and feeling of coolness of the obtained aerosol product were evaluated. The results of evaluation are shown in Table 2.

### EXAMPLE 14

An aerosol product filled with an aerosol composition for resolution of inflammation and alleviation of pain was prepared in the same manner as in Example 1 except that 10 g (20 wt%) of the concentrate of Example 3 and 40 g (80 wt%) of the liquefied petroleum gas (*1) as the liquefied gas were used. Easiness of emulsification, emulsification stability, impression from use and feeling of coolness of the obtained aerosol product were evaluated. The results of evaluation are shown in Table 2.

**TABLE 2**

| | Example | | | | | | | | | | | | | |
|---|---|---|---|---|---|---|---|---|---|---|---|---|---|---|
| | 1 | 2 | 3 | 4 | 5 | 6 | 7 | 8 | 9 | 10 | 11 | 12 | 13 | 14 |
| Easiness of emulsification | O | ⊚ | ⊚ | ○ | ⊚ | ⊚ | ○ | ⊚ | ⊚ | ○ | ○ | ⊚ | ○ | ○ |
| Emulsification stability | ⊚ | ⊚ | ⊚ | ⊚ | ⊚ | ⊚ | ○ | ⊚ | ⊚ | ○ | ○ | ⊚ | ○ | ○ |
| Impression from use | ○ | ○ | ⊚ | ○ | ○ | ⊚ | ○ | ⊚ | ⊚ | ⊚ | ⊚ | ⊚ | ○ | ○ |
| Feeling of coolness | ⊚ | ⊚ | ⊚ | ⊚ | ⊚ | ⊚ | ⊚ | ⊚ | ⊚ | ⊚ | ⊚ | ⊚ | ○ | ⊚ |

### COMPARATIVE EXAMPLES 1 to 14

Aerosol products filled with an aerosol composition for resolution of inflammation and alleviation of pain were prepared in the same manner as in Example 1 except that the concentrates shown in Table 3 were used. Easiness of emulsification, emulsification stability, impression from use and feeling of coolness of the obtained aerosol products were evaluated. The results of evaluation are shown in Table 4.

**TABLE 3**

| | Comparative Example | | | | | | |
|---|---|---|---|---|---|---|---|
| | 1 | 2 | 3 | 4 | 5 | 6 | 7 |
| Methyl salicylate | 10.0 | 10.0 | 10.0 | 10.0 | 10.0 | 10.0 | 10.0 |
| 1-Menthol | 3.0 | 3.0 | 3.0 | 3.0 | 3.0 | 3.0 | 3.0 |
| POE(20) sorbitan monolaurate (*2) | 4.0 | - | - | - | 2.0 | 2.0 | 2.0 |
| POE(60) sorbitol tetraoleate (*3) | - | - | - | - | - | - | - |
| Sorbitan monostearate (*9) | - | - | - | - | - | - | - |
| POE(40) hydrogenated castor oil (*4) | - | 4.0 | - | - | - | - | - |
| POE(10) hydrogenated castor oil (*10) | - | - | - | - | - | - | 2.0 |
| POE(2) lauryl ether (*5) | - | - | 2.0 | - | - | - | - |
| POE(20) cetyl ether (*6) | - | - | 2.0 | - | - | - | - |
| POE(40) cetyl ether (*11) | - | - | - | - | - | 2.0 | - |
| POE oleyl ether (*12) | - | - | - | - | 2.0 | - | - |
| POE·POP cetyl ether (*13) | - | - | - | 4.0 | - | - | - |
| Ethanol | 20.0 | 20.0 | 20.0 | 20.0 | 20.0 | 20.0 | 20.0 |
| Purified water | 62.0 | 62.0 | 62.0 | 62.0 | 62.0 | 62.0 | 62.0 |
| Talc | 1.0 | 1.0 | 1.0 | 1.0 | 1.0 | 1.0 | 1.0 |
| Total | 100.0 | 100.0 | 100.0 | 100.0 | 100.0 | 100.0 | 100.0 |

| 8 | 9 | 10 | 11 | 12 | 13 | 14 | |
|---|---|---|---|---|---|---|---|
| Methyl salicylate | 10.0 | 10.0 | 10.0 | 10.0 | 10.0 | 10.0 | 10.0 |
| 1-Menthol | 3.0 | 3.0 | 3.0 | 3.0 | 3.0 | 3.0 | 3.0 |
| POE(20) sorbitan monolaurate (*2) | - | - | - | - | - | - | - |
| POE(60) sorbitol tetraoleate (*3) | 4.0 | 2.0 | 2.0 | - | - | - | - |
| Sorbitan monostearate (*9) | - | - | - | 2.0 | 2.0 | 2.0 | 2.0 |
| POE(40) hydrogenated castor oil (*4) | - | - | - | 2.0 | - | - | 1.0 |
| POE(10) hydrogenated castor oil (*10) | - | - | - | - | - | - | - |
| POE(2) lauryl ether (*5) | - | - | - | - | 2.0 | - | 0.5 |
| POE(20) cetyl ether (*6) | - | - | - | - | - | - | 0.5 |
| POE(40) cetyl ether (*11) | - | - | 2.0 | - | - | - | - |
| POE oleyl ether (*12) | - | 2.0 | - | - | - | - | - |
| POE·POP cetyl ether (*13) | - | - | - | - | - | 2.0 | - |
| Ethanol | 20.0 | 20.0 | 20.0 | 20.0 | 20.0 | 20.0 | 20.0 |
| Purified water | 62.0 | 62.0 | 62.0 | 62.0 | 62.0 | 62.0 | 62.0 |
| Talc | 1.0 | 1.0 | 1.0 | 1.0 | 1.0 | 1.0 | 1.0 |
| Total | 100.0 | 100.0 | 100.0 | 100.0 | 100.0 | 100.0 | 100.0 |

| | | | | | | | |
|---|---|---|---|---|---|---|---|
| *9: NIKKOL SO-15V (trade name), HLB: 3.7, available from Nikko Chemicals Co., Ltd. *10: NIKKOL HCO-10 (trade name), HLB: 6.5, available from Nikko Chemicals Co., Ltd. *11: NIKKOL BC-40 (trade name), HLB: 20.0, available from Nikko Chemicals Co., Ltd. *12: NIKKOL BO-2 (trade name), HLB: 7.5, available from Nikko Chemicals Co., Ltd. *13: NIKKOL PBC-44 (trade name), HLB: 12.5, available from Nikko Chemicals Co., Ltd. | | | | | | | |

### COMPARATIVE EXAMPLE 15

An aerosol product filled with an aerosol composition for resolution of inflammation and alleviation of pain was prepared in the same manner as in Example 1 except that 2.5 g (5 wt%) of the concentrate of Example 3 and 47.5 g (95 wt%) of the liquefied petroleum gas (*1) as the liquefied gas were used. Easiness of emulsification, emulsification stability, impression from use and feeling of coolness of the obtained aerosol product were evaluated. The results of evaluation are shown in Table 4.

### COMPARATIVE EXAMPLE 16

An aerosol product filled with an aerosol composition for resolution of inflammation and alleviation of pain was prepared in the same manner as in Example 1 except that 35 g (70 wt%) of the concentrate of Example 3 and 15 g (30 wt%) of the liquefied petroleum gas (*1) as the liquefied gas were used. Easiness of emulsification, emulsification stability, impression from use and feeling of coolness of the obtained aerosol product were evaluated. The results of evaluation are shown in Table 4.

**TABLE 4**

| | Comparative Example | | | | | | | | | | | | | | | |
|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 |
| Easiness of emulsification | Δ | Δ | Δ | Δ | Δ | Δ | Δ | Δ | Δ | Δ | Δ | Δ | Δ | Δ | × | Δ |
| Emulsification stability | × | × | × | × | × | × | × | × | × | × | × | × | × | × | X | Δ |
| Impression from use | Δ | Δ | Δ | Δ | Δ | Δ | Δ | Δ | Δ | Δ | ×1 | ×1 | ×1 | Δ | ×2 | ×1 |
| Feeling of coolness | ○ | ○ | ○ | ○ | ○ | ○ | O | ○ | ○ | ○ | ○ | ○ | ○ | ○ | - | × |

### EXAMPLES 15 to 17

The concentrates shown in Table 5 were prepared, each solution was filled in a transparent pressure resistant polyethylene terephthalate container in an amount of 20 g (40 wt%), and an aerosol valve was fixed to the container. Then, the liquefied petroleum gas (*1) was filled as a liquefied gas in amount of 30 g (60 wt%), and the container was shaken up and down for emulsification of the concentrate and the liquefied gas to prepare an aerosol product filled with an aerosol composition for repellence of vermin. Easiness of emulsification, emulsification stability, impression from use and feeling of coolness of the obtained aerosol product were evaluated. The results of evaluation are shown in Table 6.

**TABLE 5**

| | Example 15 | Example 16 | Example 17 |
|---|---|---|---|
| N,N-diethyl-m-toluamide | 10.0 | 10.0 | 10.0 |
| 1-Menthol | 2.0 | 2.0 | 2.0 |
| POE(20) sorbitan monolaurate (*2) | 2.0 | 2.0 | 2.0 |
| POE(40) hydrogenated castor oil (*4) | 1.0 | 1.0 | 1.0 |
| POE(2) lauryl ether (*5) | 0.5 | 0.5 | 0.5 |
| POE(20) cetyl ether (*6) | 0.5 | 0.5 | 0.5 |
| Hydroxyethyl cellulose (*7) | 0.1 | - | - |
| Ethanol | 20.0 | 10.0 | 5.0 |
| Purified water | 62.9 | 73.0 | 78.0 |
| Talc | 1.0 | 1.0 | 1.0 |
| Total | 100.0 | 100.0 | 100.0 |

**TABLE 6**

| | Example 15 | Example 16 | Example 17 |
|---|---|---|---|
| Easiness of emulsification | ⊚ | ⊚ | ⊚ |
| Emulsification stability | ⊚ | ⊚ | ⊚ |
| Impression from use | ⊚ | ○ | ○ |
| Feeling of coolness | ⊚ | ⊚ | ⊚ |

### EXAMPLES 18 to 20

The concentrates shown in Table 7 were prepared, each solution was filled in a transparent pressure resistant polyethylene terephthalate container in an amount of 20 g (40 wt%), and an aerosol valve was fixed to the container. Then, the liquefied petroleum gas (*1) was filled as a liquefied gas in an amount of 30 g (60 wt%), and the container was shaken up and down for emulsification of the concentrate and the liquefied gas to prepare an aerosol product filled with an anti-sunburn aerosol composition. Easiness of emulsification, emulsification stability, impression from use and feeling of coolness of the obtained aerosol product were evaluated. The results of evaluation are shown in Table 8.

**TABLE 7**

| | Example 18 | Example 19 | Example 20 |
|---|---|---|---|
| Ethylhexyl methoxy cinnamate (*14) | 10.0 | 10.0 | 10.0 |
| Retinol | 0.5 | 0.5 | 0.5 |
| 1-Menthol | 0.5 | 0.5 | 0.5 |
| POE(20) sorbitan monolaurate (*2) | 2.0 | 2.0 | 2.0 |
| POE(40) hydrogenated castor oil (*4) | 1.0 | 1.0 | 1.0 |
| POE(2) lauryl ether (*5) | 0.5 | 0.5 | 0.5 |
| POE(20) cetyl ether (*6) | 0.5 | 0.5 | 0.5 |
| Hydroxyethyl cellulose (*7) | 0.1 | 0.1 | - |
| Ethanol | 20.0 | 10.0 | 5.0 |
| Purified water | 61.4 | 71.9 | 77.0 |
| Talc | 1.0 | 0.5 | 0.5 |
| Titanium oxide | 0.5 | 0.5 | 0.5 |
| Zinc oxide | 2.0 | 2.0 | 2.0 |
| Total | 100.0 | 100.0 | 100.0 |

| | | | |
|---|---|---|---|
| *14: Uvinul MC80N (trade name) available from BASF | | | |

**TABLE 8**

| | Example 18 | Example 19 | Example 20 |
|---|---|---|---|
| Easiness of emulsification | ⊚ | ⊚ | ⊚ |
| Emulsification stability | ⊚ | ⊚ | ⊚ |
| Impression from use | ⊚ | ○ | ○ |
| Feeling of coolness | ⊚ | ⊚ | ⊚ |

### EXAMPLES 21 and 22

The concentrates shown in Table 9 were prepared, each solution was filled in a transparent pressure resistant polyethylene terephthalate container in an amount of 25 g (50 wt%), and an aerosol valve was fixed to the container. Then, the liquefied petroleum gas (*1) was filled as a liquefied gas in an amount of 25 g (50 wt%), and the container was shaken up and down for emulsification of the concentrate and the liquefied gas to prepare an aerosol product filled with an aerosol composition for resolution of inflammation and alleviation of pain. Easiness of emulsification, emulsification stability, impression from use and feeling of coolness of the obtained aerosol products were evaluated. The results of evaluation are shown in Table 10.

### EXAMPLE 23

An aerosol product filled with an aerosol composition for resolution of inflammation and alleviation of pain was prepared in the same manner as in Example 22 except that a liquefied petroleum gas (*15) was used as the liquefied gas. Easiness of emulsification, emulsification stability, impression from use and feeling of coolness of the obtained aerosol product were evaluated. The results of evaluation are shown in Table 10.
*15: Vapor pressure at 20°C: 0.30 MPa, solubility in water at 20°C: 1 wt% or less, a mixture of propane, normal butane and isobutane

### EXAMPLE 24

An aerosol product filled with an aerosol composition for resolution of inflammation and alleviation of pain was prepared in the same manner as in Example 22 except that a gas mixture (*16) of the liquefied petroleum gas (*1) and dimethyl ether was used as the liquefied gas. Easiness of emulsification, emulsification stability, impression from use and feeling of coolness of the obtained aerosol product were evaluated. The results of evaluation are shown in Table 10.
*16: Liquefied petroleum gas / dimethyl ether = 70/30, vapor pressure at 20°C: 0.29 MPa, solubility in water at 20°C: 3.3 wt%

### COMPARATIVE EXAMPLE 17

An aerosol product filled with an aerosol composition for resolution of inflammation and alleviation of pain was prepared in the same manner as in Example 22 except that dimethyl ether (*17) was used as the liquefied gas. Easiness of emulsification, emulsification stability, impression from use and feeling of coolness of the obtained aerosol product were evaluated. The results of evaluation are shown in Table 10.
*17: Vapor pressure at 20°C: 0.42 MPa, solubility in water at 20°C: 33 wt%

**TABLE 9**

| | Example 21 | Example 22 |
|---|---|---|
| Methyl salicylate | 10.0 | 10.0 |
| 1-Menthol | 3.0 | 3.0 |
| POE(20) sorbitan monolaurate (*2) | 2.0 | 2.0 |
| POE(40) hydrogenated castor oil (*4) | 1.0 | 1.0 |
| POE(2) lauryl ether (*5) | 0.5 | 0.5 |
| POE(20) cetyl ether (*6) | 0.5 | 0.5 |
| Hydroxyethyl cellulose (*7) | 0.1 | 0.1 |
| Ethanol | 10.0 | 20.0 |
| Purified water | 71.4 | 61.8 |
| Talc | 1.0 | 1.0 |
| Perfume | 0.5 | 0.1 |
| Total | 100.0 | 100.0 |

**TABLE 10**

| | Example 21 | Example 22 | Example 23 | Example 24 | Comparative Example 17 |
|---|---|---|---|---|---|
| Easiness of emulsification | ⊚ | ⊚ | ⊚ | ○ | × |
| Emulsification stability | ⊚ | ⊚ | ⊚ | ○ | × |
| Impression from use | ⊚ | ⊚ | ⊚ | ⊚ | ×2 |
| Feeling of coolness | ⊚ | ⊚ | ⊚ | ⊚ | - |

### COMPARATIVE EXAMPLES 18 and 19

The concentrates shown in Table 11 were prepared, each solution was filled in a transparent pressure resistant polyethylene terephthalate container in an amount of 20 g (40 wt%), and an aerosol valve was fixed to the container. Then, the liquefied petroleum gas (*1) was filled as a liquefied gas in an amount of 30 g (60 wt%), and the container was shaken up and down for emulsification of the concentrate and the liquefied gas to prepare an aerosol product filled with an aerosol composition for resolution of inflammation and alleviation of pain. Easiness of emulsification, emulsification stability, impression from use and feeling of coolness of the obtained aerosol products were evaluated. The results of evaluation are shown in Table 12.

**TABLE 11**

| | Comparative Example 18 | Comparative Example 19 |
|---|---|---|
| Ethyl salicylate | 10.0 | 10.0 |
| 1-Menthol | 3.0 | 3.0 |
| POE(10) octyl phenyl ether (*18) | 4.0 | 3.0 |
| POE(3) sodium laureth sulfate (*19) | - | 1.0 |
| Hydroxyethyl cellulose (*6) | 0.1 | 0.1 |
| Ethanol | 20.0 | 20.0 |
| Purified water | 61.9 | 61.9 |
| Talc | 1.0 | 1.0 |
| Total | 100.0 | 100.0 |

| | | |
|---|---|---|
| *18: NIKKOL OP-10 (trade name), HLB: 11.5, available from Nikko Chemicals Co., Ltd. *19: NIKKOL SBL-3N-27 (trade name), anionic surfactant available from Nikko Chemicals Co., Ltd. | | |

**TABLE 12**

| | Comparative Example 18 | Comparative Example 19 |
|---|---|---|
| Easiness of emulsification | Δ | Δ |
| Emulsification stability | × | × |
| Impression from use | Δ | Δ |
| Feeling of coolness | ○ | ○ |

### EXAMPLE 25 (* not according to the invention)

The same concentrate as in Example 22 was filled in a transparent pressure resistant polyethylene terephthalate container in an amount of 10 g (20 wt%), and an aerosol valve was fixed to the container. Then, trans-1,3,3,3-tetrafluoroprop-1-ene (*20) was filled as a liquefied gas in an amount of 40 g (80 wt%), and the container was shaken up and down for emulsification of the concentrate and the liquefied gas to prepare an aerosol product filled with an aerosol composition for resolution of inflammation and alleviation of pain. Easiness of emulsification, emulsification stability, impression from use and feeling of coolness of the obtained aerosol product were evaluated. The results of evaluation are shown in Table 13.
*20: Vapor pressure at 20°C: 0.42 MPa, solubility in water at 20°C: 1 wt% or less

### EXAMPLE 26 (* not according to the invention)

An aerosol product filled with an aerosol composition for resolution of inflammation and alleviation of pain was prepared in the same manner as in Example 25 except that 15 g (30 wt%) of the same concentrate as in Example 22 and 35 g (70 wt%) of trans-1,3,3,3-tetrafluoroprop-1-ene (*20) as the liquefied gas were filled. Easiness of emulsification, emulsification stability, impression from use and feeling of coolness of the obtained aerosol product were evaluated. The results of evaluation are shown in Table 13.

### COMPARATIVE EXAMPLE 20

An aerosol product filled with an aerosol composition for resolution of inflammation and alleviation of pain was prepared in the same manner as in Example 25 except that 35 g (70 wt%) of the same concentrate as in Example 22 and 15 g (30 wt%) of trans-1,3,3,3-tetrafluoroprop-1-ene (*20) as the liquefied gas were filled. Easiness of emulsification, emulsification stability, impression from use and feeling of coolness of the obtained aerosol product were evaluated. The results of evaluation are shown in Table 13.

**TABLE 13**

| | Example 25* | Example 26* | Comparative Example 20 |
|---|---|---|---|
| Easiness of emulsification | ○ | ○ | × |
| Emulsification stability | ○ | ○ | × |
| Impression from use | ○ | ○ | × |
| Feeling of coolness | ○ | ○ | × |

| | | | |
|---|---|---|---|
| * Not according to the invention | | | |

### EXAMPLES 27 and 28

The concentrates shown in Table 14 were prepared, each solution was filled in a transparent pressure resistant polyethylene terephthalate container in an amount of 20 g (40 wt%), and an aerosol valve was fixed to the container. Then, the liquefied petroleum gas (*1) was filled as a liquefied gas in an amount of 30 g (60 wt%), and the container was shaken up and down for emulsification of the concentrate and the liquefied gas to prepare an aerosol product filled with an aerosol composition for resolution of inflammation and alleviation of pain. Easiness of emulsification, emulsification stability, impression from use and feeling of coolness of the obtained aerosol products were evaluated. The results of evaluation are shown in Table 15.

**TABLE 14**

| | Example 27 | Example 28 |
|---|---|---|
| Felbinac | 3.0 | 3.0 |
| 1-Menthol | 3.0 | 3.0 |
| POE(20) sorbitan monolaurate (*2) | 2.0 | 2.0 |
| POE(40) hydrogenated castor oil (*4) | 1.0 | 1.5 |
| Hydroxyethyl cellulose (*7) | 0.1 | 0.1 |
| Ethanol | 15.0 | 20.0 |
| Purified water | 72.3 | 66.8 |
| Talc | 0.5 | 0.5 |
| Perfume | 0.1 | 0.1 |
| Triethanolamine | 3.0 | 3.0 |
| Total | 100.0 | 100.0 |

**TABLE 15**

| | Example 27 | Example 28 |
|---|---|---|
| Easiness of emulsification | ○ | ○ |
| Emulsification stability | ○ | ○ |
| Impression from use | ○ | ○ |
| Feeling of coolness | ⊚ | ⊚ |

### EXAMPLES 29 and 30

The concentrates shown in Table 16 were prepared, each solution was filled in a transparent pressure resistant polyethylene terephthalate container in an amount of 20 g (40 wt%), and an aerosol valve was fixed to the container. Then, the liquefied petroleum gas (*1) was filled as a liquefied gas in an amount of 30 g (60 wt%), and the container was shaken up and down for emulsification of the concentrate and the liquefied gas to prepare an aerosol product filled with an aerosol composition for refreshing. Easiness of emulsification, emulsification stability, impression from use and feeling of coolness of the obtained aerosol products were evaluated. The results of evaluation are shown in Table 17.

**TABLE 16**

| | Example 29 | Example 30 |
|---|---|---|
| 1-Menthol | 5.0 | 10.0 |
| POE(20) sorbitan monolaurate (*2) | 2.0 | 2.0 |
| POE(40) hydrogenated castor oil (*4) | 1.0 | 1.0 |
| POE(2) lauryl ether (*5) | 0.5 | 0.5 |
| POE(20) cetyl ether (*6) | 0.5 | 2.0 |
| Hydroxyethyl cellulose (*7) | 0.1 | 0.1 |
| Isopropyl myristate (*21) | - | 10.0 |
| Ethanol | 20.0 | 20.0 |
| Purified water | 69.8 | 53.3 |
| Talc | 1.0 | 1.0 |
| Perfume | 0.1 | 0.1 |
| Total | 100.0 | 100.0 |

| | | |
|---|---|---|
| *21: IPM-EX (trade name) available from Nikko Chemicals Co., Ltd. | | |

**TABLE 17**

| | Example 29 | Example 30 |
|---|---|---|
| Easiness of emulsification | Δ | ○ |
| Emulsification stability | ○ | ⊚ |
| Impression from use | ○ | ⊚ |
| Feeling of coolness | ⊚ | ⊚ |

## Claims

1. A foamable aerosol composition comprising a concentrate and a liquefied gas, wherein
the concentrate comprises active ingredients, surfactants, a monovalent alcohol having 2 to 3 carbon atoms and water,
the active ingredients are poorly water-soluble active ingredients having a solubility in water at 25°C of less than 10 wt%.
the surfactants comprise polyoxyethylene-added fatty acid ester and a nonionic surfactant having HLB of 8 to 19,
wherein the polyoxyethylene-added fatty acid ester is polyoxyethylene sorbitan fatty acid ester, and the nonionic surfactant is polyoxyethylene hydrogenated castor oil and/or an ether surfactant,
the liquefied gas is a poorly water-soluble liquefied gas having solubility in water at 20°C of less than 10wt%,
the liquefied gas contains aliphatic hydrocarbon in an amount of 60 wt% or more,
the ratio of the concentrate to the liquefied gas is 60/40 to 10/90 (weight ratio), the amount of the poorly water-soluble active ingredients is from 5 to 30 wt% in the concentrate, and
the concentrate and the liquefied gas are emulsified.

2. The foamable aerosol composition of claim 1, wherein the poorly water-soluble active ingredients are antiphlogistic anodynes.

3. The foamable aerosol composition of claim 1, wherein the amount of the monovalent alcohol having 2 to 3 carbon atoms is from 5 to 40 wt% in the concentrate.

4. The foamable aerosol composition of claim 1, wherein the amount of water is from 40 to 85 wt% in the concentrate.

## Patentansprüche

1. Schäumbare Aerosolzusammensetzung, umfassend ein Konzentrat und ein verflüssigtes Gas, wobei
das Konzentrat aktive Inhaltsstoffe, Tenside, einen einwertigen Alkohol, der 2 bis 3 Kohlenstoffatome aufweist und Wasser umfasst,
die aktiven Inhaltsstoffe schlecht wasserlösliche aktive Inhaltsstoffe, die eine Wasserlöslichkeit bei 25 °C von weniger als 10 Gew.-% aufweisen, sind,
die Tenside Polyoxyethylen-addierten Fettsäureester und ein nichtionisches Tensid umfassen, das HLB von 8 bis 19 aufweist,
wobei der Polyoxyethylen-addierte Fettsäureester Polyoxyethylen-Sorbitan Fettsäureester ist und das nichtionische Tensid Polyoxyethylen-hydriertes Rizinusöl und/oder ein Ethertensid ist,
das verflüssigte Gas ein schlecht wasserlösliches verflüssigtes Gas ist, das eine Wasserlöslichkeit bei 20 °C von weniger als 10 Gew.-% aufweist,
das verflüssigte Gas aliphatischen Kohlenwasserstoff in einer Menge von 60 Gew.-% oder mehr enthält,
das Verhältnis des Konzentrats zum verflüssigten Gas 60/40 bis 10/90 (Gewichtsverhältnis) ist,
die Menge der schlecht wasserlöslichen aktiven Inhaltsstoffe von 5 bis 30 Gew.-% im Konzentrat ist, und
das Konzentrat und das verflüssigte Gas emulgiert sind.

2. Schäumbare Aerosolzusammensetzung nach Anspruch 1, wobei die schlecht wasserlöslichen aktiven Inhaltsstoffe antiphlogistische Anodyne sind.

3. Schäumbare Aerosolzusammensetzung nach Anspruch 1, wobei die Menge des einwertigen Alkohols, der 2 bis 3 Kohlenstoffatome aufweist, 5 bis 40 Gew.-% im Konzentrat ist.

4. Schäumbare Aerosolzusammensetzung nach Anspruch 1, wobei die Menge von Wasser 40 bis 85 Gew.-% im Konzentrat ist.

## Revendications

1. Composition d'aérosol moussante comprenant un concentré et un gaz liquéfié, dans laquelle
le concentré comprend des ingrédients actifs, des tensioactifs, un alcool monovalent ayant 2 à 3 atomes de carbone et de l'eau,
les ingrédients actifs sont des ingrédients actifs faiblement solubles dans l'eau ayant une solubilité dans l'eau à 25 °C de moins de 10 % en poids,
les tensioactifs comprennent de l'ester d'acide gras additionné de polyoxyéthylène et un tensioactif non ionique ayant un HLB de 8 à 19,
dans lequel l'ester d'acide gras additionné de polyoxyéthylène est de l'ester d'acide gras de polyoxyéthylène sorbitane, et le tensioactif non ionique est de l'huile de ricin hydrogénée de polyoxyéthylène et/ou un tensioactif d'éther,
le gaz liquéfié est un gaz liquéfié faiblement soluble dans l'eau ayant une solubilité dans l'eau à 20 °C de moins de 10 % en poids,
le gaz liquéfié contient un hydrocarbure aliphatique en une quantité de 60 % en poids ou plus,
le rapport du concentré sur le gaz liquéfié est de 60/40 à 10/90 (rapport en poids), la quantité des ingrédients actifs faiblement solubles dans l'eau est de 5 à 30 % en poids dans le concentré, et
le concentré et le gaz liquéfié sont émulsifiés.

2. Composition d'aérosol moussante selon la revendication 1, dans laquelle les ingrédients actifs faiblement solubles dans l'eau sont des anodynes antiphlogistiques.

3. Composition d'aérosol moussante selon la revendication 1, dans laquelle la quantité de l'alcool monovalent ayant 2 à 3 atomes de carbone est de 5 à 40 % en poids dans le concentré.

4. Composition d'aérosol moussante selon la revendication 1, dans laquelle la quantité d'eau est de 40 à 85 % en poids dans le concentré.
